# EUROPEAN PATENT SPECIFICATION

(11) **EP 1 631 330 B1**
(45) Date of publication and mention of the grant of the patent: **25.06.2014**
(21) Application number: 04753498.7
(22) Date of filing: 27.05.2004
(51) Int. Cl.: A61L 15/46, A61F 13/84, A61F 13/511

(54) **COMPOSITION AND PROCESS FOR COATING A SUBSTRATE**
ZUSAMMENSETZUNGEN UND VERFAHREN ZUR BESCHICHTUNG EINER LAGE
COMPOSITION ET PROCEDE PERMETTANT DE REVETIR UN SUBSTRAT

(30) Priority: 30.05.2003 US 474485 P
(43) Date of publication of application: 08.03.2006
(73) Proprietor: THE PROCTER & GAMBLE COMPANY, Cincinnati, OH 45202 (US)
(72) Inventor: CIELENSKI, Peter, Ryan, Westchester, OH 45069 (US); MCCUSKER, Henry, William, III, Cincinnati, OH 45230 (US)
(74) Representative: Kremer, Véronique Marie Joséphine
(86) International application number: PCT/US2004/016679
(87) International publication number: WO 2004/108177

(56) References cited:
- WO-A-98/26808
- WO-A-98/27261
- DE-A- 4 011 928
- US-A1- 2002 164 295

## Description

### FIELD OF INVENTION

The present invention relates to compositions, processes incorporating the compositions, and articles (such as substrates, catamenials, diapers, pantiliners, training pants, adult incontinence undergarments, etc.) made by the process, which release perfume and subsequently are able to minimize odor caused from bodily fluids through the incorporation of a starch encapsulated accord (SEA) and a carrier.

### BACKGROUND OF THE INVENTION

A wide variety of fluid absorbent structures known in the art absorb body fluids such as blood, urine, menses, and the like, and are sanitary and comfortable in use. Disposable products of this type generally comprise fluid-permeable topsheet material, fluid absorbent core, and fluid-impermeable backsheet material. Various shapes, sizes and thicknesses of such articles have been explored in an attempt to make their use more comfortable and convenient.

Odor control in absorbent products has been under investigation for many years. Many bodily fluids have an unpleasant odor, or develop such odors when in contact with air and/or bacteria for prolonged periods. Consumers typically use malodor to determine the need for change of a diaper, catamenial and the like. Malodor is also an integral component of the toilet training process.

One alternative to relying on body malodor as one factor to indicate the need to change is through the use of a "scent signal." A "scent signal" is a positive perfume odor which signals to a consumer the need to at least inspect, if not remove the absorbent product. Typically, the scent signal is released when contacted by bodily fluids, such as sweat, urine, menses and the like. WO 98/26808 discloses absorbent articles with an odour control system.

One material which is suitable for incorporation into absorbent products to generate a scent signal are the starch encapsulated accords, or SEA. SEAs are solid particles comprising water-soluble cellular matrixes containing perfume stably held in the cells. When SEAs are contacted with water, such as moisture, urine etc, the water-soluble cellular matrix at least partially dissolves thereby allowing for the perfumes release, thereby generating a scent signal.

However, while it would be advantageous to incorporate SEAs into absorbent products to provide a scent signal, there are numerous problems associated with their manufacture. Typically, these problems are related to preventing or minimizing the SEAs exposure to moisture and reducing and/or eliminating the potential for the SEAs to generate dust while still providing the SEAs in form which is stable, cheap, and easy to incorporate into process for the manufacture of a substrate, such as those incorporated into absorbent products. Another problem associated with incorporation of a SEA onto a substrate is that of accuracy, namely it is difficult and costly to be able to deliver SEA to a substrate with any degree of reasonable accuracy required for a commercial process.

### SUMMARY OF THE INVENTION

The present invention provides a process for the delivery of a composition on to a substrate comprising:
(a) providing said coating composition, the coating composition comprising:
   (i) a starch encapsulated accord; and
   (ii) a carrier; selected from the group consisting of alkoxylated nonionic surfactants, mineral oil, polyols, paraffin waxes and combinations thereof.
   wherein the weight ratio of the carrier to the starch encapsulated accord is equal to or greater than about 1:1; and
(b) delivering and attaching an effective amount of the coating composition to the substrate.

It should be understood that every limit given throughout this specification will include every lower or higher limit, as the case may be, as if such lower or higher limit was expressly written herein. Every range given throughout this specification will include every narrower range that falls within such broader range, as if such narrower ranges were all expressly written herein.

The citation of any document is not to be construed as an admission that it is prior art with respect to the present invention. All percentages, ratios and proportions are by weight, and all temperatures are in degrees Celsius (°C), unless otherwise specified. All measurements are in SI units unless otherwise specified.

### BRIEF DESCRIPTION OF THE DRAWINGS

The above-mentioned and other features and objects of this invention and the manner of attaining them will become more apparent, and the invention itself will be better understood, by reference to the following description of the invention taken in conjunction with the accompanying drawings, wherein:
Figure 1 is a perspective view of one possible apparatus suitable for use in the process of the present invention.
Figure 2 is a perspective view of a portion of the apparatus of Figure 1 illustrating when the angle θ of the applicator 80 relative to reference line 100 is approximately minus 45°.
Figure 3 is another perspective view of a portion of the apparatus of Figure 1 illustrating when the angle θ of the applicator 80 relative to reference line 100 is approximately 0°.
Figure 4 is a perspective view of a portion of the apparatus of Figure 1 illustrating when the angle θ of the applicator 80 relative to reference line 100 is approximately 45°.

### DETAILED DESCRIPTION OF THE INVENTION

As used herein, the term "comprising" means that the various components, ingredients, or steps, can be conjointly employed in practicing the present invention. Accordingly, the term "comprising" is open-ended and encompasses the more restrictive terms "consisting essentially of' and "consisting of." Other terms may be defined as they are discussed in greater detail herein.

The process of the present invention comprises the steps of providing, delivering and attaching an effective amount of a coating composition, as described herein, to a substrate, also described herein.

### (a) Coating Composition

The coating composition of the present invention has two essential components, a starch encapsulated accord, or SEA, and a carrier. These two components are present in a weight ratio of carrier to SEA of equal to or greater than 1:1. In one optional embodiment of the present invention the coating composition comprises from about 0% to about 10%, more preferably from about 0% to about 5% by weight of coating composition of water. In one highly preferred embodiment of the present invention the coating composition is essentially free from water and contains only trace amounts of water. This low water content provides added stability and extended life to the SEAs, while also reducing manufacturing costs due to reduced product loss.

The coating compositions of the present invention may comprise optional ingredients, such as but not limited to, aesthetic components, pigments and the like. Some illustrative optional ingredients are described herein.

Desirably, at least an effective amount of the coating composition is applied to the article. Effective amounts are typically those which provide a noticeable scent signal to the consumer to signify the substrate on which the coating composition is attached has been contacted with sufficient aqueous fluid (e.g., menses, urine, etc.) or water containing solid (e.g., feces). In one optional embodiment of the present invention when the substrate is a part of a disposable absorbent article the typical amount of the coating composition present on the substrate is from about 0.001 g to about 5 g, preferably from about 0.005 g to about 1 g, more preferably from about 0.01 g to about 0.5 g, per substrate.

### (i) Starch Encapsulated Accord (or SEA)

The coating compositions of the present invention comprise a starch encapsulated accord, or SEA. SEAs are solid particles comprising water-soluble cellular matrixes containing perfume stably held in the cells. Typically, the SEAs comprise: (i) perfume preferably ranging from about 20% to about 60%, more preferably from about 20% to about 50%, by weight of the SEA; (ii) mainly polysaccharide and/or polyhydroxy compounds, preferably from at least 20%, more preferably from about 50% to about 80% by weight of the SEA; and (iii) optional adjunct ingredients ranging from about 0% to about 5%, such as but not limited to wetting agents, process aids, flow agent and the like and combinations thereof.

SEAs comprise mainly polysaccharide and polyhydroxy compounds. The polysaccharides are preferably higher polysaccharides of the non-sweet, colloidally-soluble types, such as natural gums, e.g., gum arabic, starch derivatives, dextrinized and hydrolyzed starches, and the like. The polyhydroxy compounds are preferably alcohols, plant-type sugars, lactones, monoethers, and acetals. The SEAs useful in the present invention are typically prepared by forming an aqueous phase of the polysaccharide and polyhydroxy compound in proper proportions with added emulsifier if necessary or desirable, emulsifying the perfumes in the aqueous phase, and removing moisture while the mass is plastic or flowable (e.g., by spray drying droplets of the emulsion). The SEAs and process details are disclosed in, e.g., U.S. Pat. No. 3,971,852, Brennner et al., issued Jul. 27, 1976.

In one optional embodiment of the present invention it is desirable to have only minimal non-encapsulated surface perfume, more preferably of less than about 1% by weight of the SEAs.

In another optional preferred embodiment of the present invention the SEAs preferably have a particle size of from about 0.5 µm to about 1000 µm and an average particle size of from about 1 µm to about 300 µm, more preferably a particle size of from about 1 µm to about 500 µm and an average particle size of from about 1 µm to about 100 µm, and even more preferably a particle size of from about 1 µm to about 100 µm and an average particle size of from about 10 µm to about 50 µm.

SEAs can be obtained commercially, e.g., as IN-CAP® from Polak's Frutal Works, Inc., Middletown, N.Y.; and as Optilok System® encapsulated perfumes from Encapsulated Technology, Inc., Nyack, N.Y. Other suitable SEAs are available from Haarmann & Reimer, Teterboro, NJ USA,

The perfume ingredients and compositions of this invention are conventional and well known in the art. Selection of any perfume component, or amount of perfume, is based on functional and aesthetic considerations. Preferred perfume components useful in the present invention are highly volatile and moderately volatile perfume ingredients, more preferably highly volatile, low boiling ingredients.

The highly volatile, low boiling, perfume ingredients typically have boiling points of about 250 °C or lower. These highly volatile perfume ingredients are fleeting and are quickly lost as they are released. Many of the more moderately volatile perfume ingredients are also quickly lost. The moderately volatile perfume ingredients are those having boiling points of from about 250 °C. to about 300 °C. Many of the perfume ingredients as discussed hereinafter, along with their odor characters, and their physical and chemical properties, such as boiling point and molecular weight, are given in "Perfume and Flavor Chemicals (Aroma Chemicals)," Steffen Arctander, published by the author, 1969.

Examples of the highly volatile, low boiling, perfume ingredients are: anethole, benzaldehyde, benzyl acetate, benzyl alcohol, benzyl formate, iso-bomyl acetate, camphene, cis-citral (neral), citronellal, citronellol, citronellyl acetate, para-cymene, decanal, dihydrolinalool, dihydromyrcenol, dimethyl phenyl carbinol, eucalyptol, geranial, geraniol, geranyl acetate, geranyl nitrile, cis-3-hexenyl acetate, hydroxycitronellal, d-limonene, linalool, linalool oxide, linalyl acetate, linalyl propionate, methyl anthranilate, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, phenyl ethyl alcohol, alpha-pinene, beta-pinene, gamma-terpinene, alpha-terpineol, beta-terpineol, terpinyl acetate, and vertenex (para-tertiary-butyl cyclohexyl acetate). Some natural oils also contain large percentages of highly volatile perfume ingredients. For example, lavandin contains as major components: linalool; linalyl acetate; geraniol; and citronellol. Lemon oil and orange terpenes both contain about 95% of d-limonene.

Examples of moderately volatile perfume ingredients are: amyl cinnamic aldehyde, iso-amyl salicylate, beta-caryophyllene, cedrene, cinnamic alcohol, coumarin, dimethyl benzyl carbinyl acetate, ethyl vanillin, eugenol, iso-eugenol, flor acetate, heliotropine, 3-cis-hexenyl salicylate, hexyl salicylate, lilial (para-tertiarybutyl-alpha-methyl hydrocinnamic aldehyde), gamma-metyhyl ionone, nerolidol, patchouli alcohol, phenyl hexanol, beta-selinene, trichloromethyl phenyl carbinyl acetate, triethyl citrate, vanillin, and veratraldehyde. Cedarwood terpenes are composed mainly of alpha-cedrene, beta-cedrene, and other C₁₅ H₂₄ sesquiterpenes.

An example of an SEA is an IN-CAP microcapsule sample, obtainable from Polak's Frutal Works, Inc., having about 50% perfume loading and particle size range of from about 3 microns to about 100 microns. Major components of the perfume are highly volatile components, such as citral and d-limonene.

Typically the SEA is present in the coating composition in an effective amount, namely an amount which is effective to provide for effective mixing of the SEA and carrier, as well as to enable the coating composition to be delivered and attached to a substrate. It is preferred that the SEA is present in the coating compositions at levels of preferably from about 0.01% to about 99%, more preferably from about 0.5% to about 97%, and more preferably from about 1.0% to about 98%, by weight of the coating composition.

### (ii) Carrier

The coating compositions of the present invention comprise a carrier, which must be capable of suspending the SEAs while having minimal or preferably no interaction with the SEAs which cause the perfume to be released. It is preferred that the carrier is present in the coating compositions at levels of preferably from about 0.01% to about 99%, more preferably from about 0.5% to about 97 %, and more preferably from about 1.0% to about 98%, by weight of the coating composition.

The carrier may be a liquid or it may be a solid which is a liquid at the temperature which the process is performed.

Suitable carriers include Polyalkoxylated materials having a weight average molecular weight of from about 200 to about 20,000 g/mole and the weight percent of the polyalkoxy portion being from about 50% to about 99%. Specific examples include: Tetronic® and Tetronic R®; and Varstat 66®. Tetronic® and Tetronic R® are block copolymeric surfactants, manufactured by BASF Corporation. Varstat 66® is sold by Sherex Chemical Company.

In one optional embodiment of the present invention the carrier may be an oil, which is liquid or in the molten phase at the temperature which the process is performed (i.e. a solid which is liquid at temperatures at which the process is to be performed). Suitable oils include but are not limited to, mineral oil, light oil, white mineral oil, vaseline, liquid petroleum, petrolatum, petrolatum gel and combinations thereof. Other materials suitable for use as carriers include, but are not limited to, paraffin waxes, fatty alcohols, such as but not limited to stearyl alcohol and the like, and combinations thereof.

In one optional embodiment the carrier is selected from the group consisting of alkoxylated nonionic surfactants, mineral oil, polyols, paraffin waxes, and combinations thereof.

It is also within the scope of the present invention for carrier to comprise a mixture of possible carriers.

Regardless of which carrier is to be used, it must be compatible with the SEA and suitable for the intended use of the substrate to which it is attached. For example if the substrate is to be incorporated into the topsheet of a baby diaper, the carrier needs to be compatible for use in a diaper.

### Ratio

The weight ratio of the carrier to the SEA may be equal to or greater than about 1:1, preferably from about 1:1 to about 10:1, even more preferably from equal to or greater than about 1:1 to about 5:1. It has been surprisingly found that this ratio is critical to ensure the even suspension of the SEA in the carrier as well as improving the processability, delivery and attachment of the coating composition to a substrate.

This relative relationship (i.e. the ratio, between the carrier and SEA) provides a coating composition which is easy to process and provides simple delivery and good attachment to the substrate. The relationship between the carrier and SEA also allows for effective delivery of the optimal amount of SEA to a substrate thereby producing cost savings in raw materials, and reducing losses of SEA during the various process steps. The coating compositions of the present invention also are highly processable allowing for efficient and simplified delivery of the SEA, in the coating composition, to the substrate. For example, since the coating composition is readily processable, the SEAs can be accurately targeted for deposition onto a substrate (e.g. the deposition of the coating composition can be easily limited to one or more regions on the substrate without simply coating the entire substrate). This is an additional cost saving as the targeted deposition of SEA means it is possible to reduce the amount of SEA necessary, thereby further reducing costs.

### (iii) Optional Ingredients

The coating compositions used in the present invention may optionally contain one or more optional ingredients. Examples of these ingredients include, but are not limited to: aesthetic components, pigments, colorings, colorants, anti-caking agents, antifoaming agents, preservative, dye, antimicrobial agents (e.g., quaternium-15, methyl paraben, ethyl paraben, propyl paraben, DMDM hydantoin, Suttocide A, IPBC, etc.), antioxidants, fluorescence agents, binders, fumed silica, biological additives, buffering agents, bulking agents, chelating agents, chemical additives, solvents (other than water), cosmetic biocides, denaturants, humectants, opacifying agents, pH adjusters, process aids, reducing agents, sequestrants, binders, thickeners, hydrocolloids, zeolites, and the like.

Optional ingredients, when present, are each typically employed in compositions at levels of from about 0.0001% to about 99.9%, preferably from about 0.001% to about 99%, and more preferably from about 0.01% to about 97%, by weight of the coating composition.

### (b) Delivery and Attaching

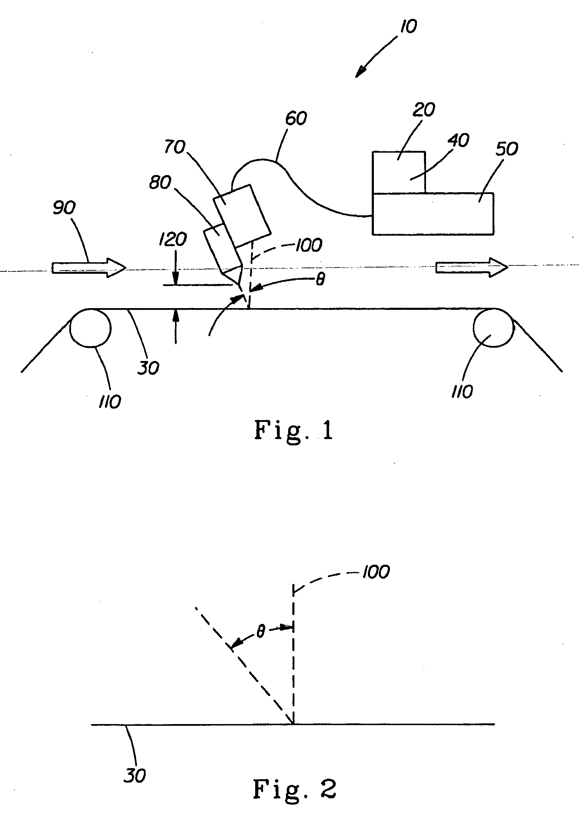

Figure 1 illustrates one embodiment of the process of the present invention, namely the process 10 for the delivery of a coating composition 20 to a substrate 30. The coating composition 20 as described herein, comprises a carrier, which is either liquid or liquid at the temperature the process 10 is performed, and SEA particles, is provided in storage tank 40. The carrier and SEA may be delivered to the storage tank 40 already mixed together in the appropriate ratio, or alternatively they may be mixed together in storage tank 40. In an alternative embodiment not shown, the carrier and SEA are stored in separate compartments of storage tank 40 and mixed together to form the coating composition 20 when dispensed from the storage tank 40. Storage tank 40 may optionally include a heater to maintain the coating compositions 20, or components thereof, at a specific temperature, i.e. to keep the carrier liquid, or to heat or preheat the coating composition 20. Suitable storage tanks include, but are not limited to, the DX Melter available from Nordson Corporation Dawsonville, Georgia USA, or the Dynamelt S available from ITW Dynatec AMERICAS, Hendersonville, TN USA.

The coating composition 20 is then moved from the storage tank 40 to supply tank 50. This movement may be achieved by any suitable conventional process, such as pumping, gravity feed etc, with the use of a pump being preferred. Supply tank 50 has temperature control to ensure that the coating composition is at the desired temperature when it leaves the supply tank 50 via delivery hose 60. Supply tank 50, may also be used to ensure even and consistent mixing of the coating composition 20. Delivery hose 60 is preferably at least insulated, more preferably insulated and temperature controlled, so the coating composition 20 remains at the desired process temperature. Suitable supply tanks include, but are not limited to, the DX Melter available from Nordson Corporation Dawsonville, Georgia USA, or the Dynamelt S available from ITW Dynatec AMERICAS, Hendersonville, TN USA. Suitable delivery hoses include, but are not limited to, the Advanced Technology RTD Hoses available from Nordson Corporation Dawsonville, Georgia USA, or the Dynaflex Hot Melt Adhesive Hose available from ITW Dynatec AMERICAS, Hendersonville, TN USA.

Once the coating composition 20 passes from the supply tank 50 and through the delivery hose 60 it arrives at placement-controlled applicator 70. The placement-controlled applicator 70 is also preferably at least insulated, more preferably insulated and temperature controlled, so the coating composition 20 remains at the desired process temperature. The placement-controlled applicator 70 supplies the coating composition 20 to the applicator 80 which in turn delivers the coating composition 20 to substrate 30.

Applicator 80 may be any suitable applicator, especially those which are typically used for applying heated liquids to substrates, such as but not limited to, one or more bead extruders, slot die coaters, spray nozzles and the like and combinations thereof. The applicator 80 may comprise one or more of these, which may be arranged parallel across the width of the web, or arranged in series. In any event, whichever applicator is selected, it must be capable of applying a liquid, preferably a heated liquid, containing suspended particles on to a substrate which may be moving. In one embodiment of the present invention the applicator has a dimension, i.e. width, length circumference etc., which is greater than about 200 µm more preferably from about 400 µm to about 1000 µm.

Suitable slot die coaters include, but are not limited to, the EP11 Applicator available from Nordson Corporation Dawsonville, Georgia USA, or the MR1300 Slot Die Coater available from ITW Dynatec AMERICAS, Hendersonville, TN USA. Suitable bead coaters include, but are not limited to, the H203 Applicator available from Nordson Corporation Dawsonville, Georgia USA.

In one optional embodiment of the present invention the applicator 80 is a bead nozzle. The bead nozzles preferably have a circular or elliptical cross-sectional shape. Typically, the applicator 80 will comprise at least one, preferably one or two nozzles. The size of the cross-sectional shape of the bead nozzle will depend upon many factors, including but not limited to, size of SEA particles, muzzle velocity, desired flow rate, etc. The preferred circular or elliptical bead nozzles typically have diameters in the range of from greater than about 200 µm more preferably from about 400 µm to about 1000 µm.

In another optional embodiment of the present invention the applicator 80 is a slot die coater. Typically, the applicator 80 will comprise at least one, preferably between one and ten slots. The size of the slots of the slot die coater will depend upon many factors, including but not limited to, size of SEA particles, muzzle velocity, desired flow rate, etc. Typically, the slots will be square or rectangular and have a width and a length of equal to or greater than about 200 µm. In one preferred embodiment each slot of the slot die coater is rectangular having a length of from about 200 µm to about 250 mm, and a width of from about 200 µm to about 80 mm.

In Figure 1, the substrate 30 is shown moving in machine direction 90, however the substrate may be stationary relative to the applicator 80 when the coating composition 20 is delivered thereto, i.e., both the substrate 30 and applicator 80 are stationary, or both are moving at the same velocity. The velocity of the substrate 30 relative to the applicator 80 will depend upon may factors, such as but not limited to, substrate, coating composition, muzzle velocity of the coating composition 20, impact velocity of the coating composition 20 on the substrate 30, applicator type and the like and combinations thereof. In one alternative embodiment the substrate 30 is moving at a velocity of from about 0 to about 6 meters per second relative to the applicator 80. This embodiment is illustrated in Figure 1, where the applicator 80 is stationary and rollers 110 move substrate 30 in machine direction 90.

The rate at which the coating composition 20 is delivered to the substrate 30 will depend upon may factors, such as but not limited to, substrate, coating composition, amount of coating composition desired on substrate (e.g., grams of coating composition per square meter of substrate), muzzle velocity of the coating composition 20, impact velocity of the coating composition 20 on the substrate 30, applicator type and the like and combinations thereof.

In one optional embodiment of the present invention coating composition 20 is delivered to the substrate 30 at a rate of from about 1 to about 500 grams of coating composition per minute. In one more preferred embodiment of the present invention when applicator 80 is a bead extruder, coating composition 20 is delivered to the substrate 30 at a rate of from about 30 to about 120 grams of coating composition per minute per nozzle. In another more preferred embodiment of the present invention when applicator 80 is a slot die coater, coating composition 20 is delivered to the substrate 30 at a rate of from about 35 to about 230 grams of coating composition per minute.

The coating composition 20 may be delivered to the substrate either continuously or intermittently. In one optional embodiment the coating composition 20 is delivered to the substrate 30 at a frequency of from about 0 (i.e., continuous delivery) to about 50 Hz, more preferably from about 0 to about 20 Hz, even more preferably from about 0 to about 15 Hz.

The distance 120 from the applicator 80 to the substrate 30 will depend upon many factors, including but not limited to substrate, coating composition, amount of coating composition desired on substrate (e.g., grams of coating composition per square meter of substrate), muzzle velocity of the coating composition 20, impact velocity of the coating composition 20 on the substrate 30, applicator type, temperature of the coating composition 20 and the like and combinations thereof. It is preferred that the distance 120 of the applicator 80 from the substrate 30 be from about 1 mm to about 100 mm, preferably from about 5 mm to about 50 mm.

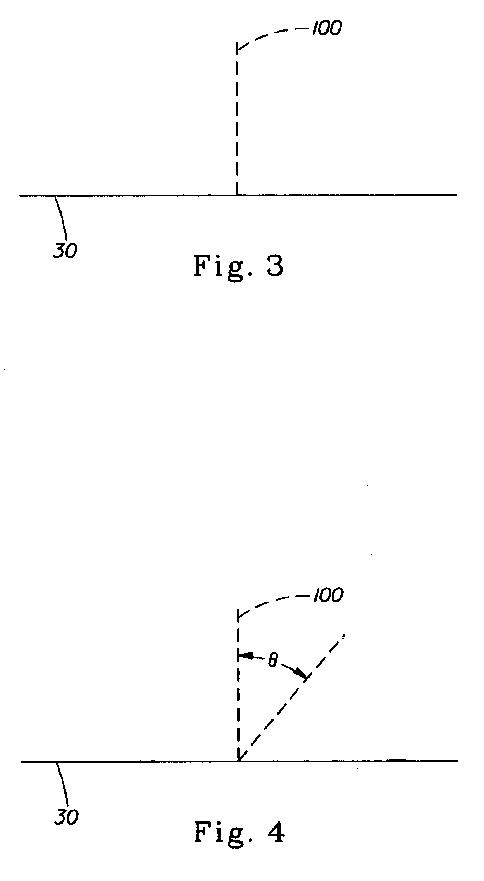

In Figure 1, the angle θ, is the angle of the applicator 80 relative to the reference line 100 which is perpendicular, i.e. 90° to the substrate 30. The angle θ will depend upon many factors, such as but not limited to, substrate, coating composition, amount of coating composition desired on substrate (e.g., grams of coating composition per square meter of substrate), muzzle velocity of the coating composition 20, impact velocity of the coating composition 20 on the substrate 30, applicator type and the like and combinations thereof. Typically, the angle θ will range from about minus 60° to about 60°, more preferably from about minus 45° to about 45°. Figure 2 illustrates one embodiment where the angle θ is about minus 45°. Figure 3 illustrates one embodiment where the angle θ is about 0°. And lastly, Figure 4 illustrates one embodiment where the angle θ is about 45°.

The process of the present invention may be performed at any suitable temperature. The temperature at which the process is performed at will depend upon may factors, such as but not limited to, the carrier used, the SEA used, substrate used, desired viscosity of the coating composition, amount of coating composition desired on substrate (e.g. grams of coating composition per square meter of substrate), muzzle velocity of the coating composition 20, impact velocity of the coating composition 20 on the substrate 30, applicator type and the like and combinations thereof. In one optional embodiment of the present invention the process is performed at a temperature greater than the melting point of the carrier. In another the embodiment of the present invention the process is performed at ambient temperature, typically from about 15°C to about 35°C. In yet another alternative embodiment the process is preferably performed in a temperature range of from about 20°C to about 110°C, more preferably from about 30°C to about 110°C, even more preferably from about 55°C to about 105°C.

### Substrate

The process of the present invention delivers and attaches an effective amount of a coating composition, described herein, to a substrate. In one optional embodiment of the present invention the substrate is selected from a group consisting of nonwoven material, film, woven material and combinations thereof. The substrate may also include laminates comprising two or more layers of materials.

The nonwoven material, and woven material may comprise fibers made by nature (natural fibers), made by man (synthetic or man-made), or combinations thereof. Example natural fibers include but are not limited to: animal fibers such as wool, silk, fur, and hair; vegetable fibers such as cellulose, cotton, flax, linen, and hemp; and certain naturally occurring mineral fibers. Synthetic fibers can be derived from natural fibers or not. Example synthetic fibers which are derived from natural fibers include but are not limited to rayon and lyocell, both of which are derived from cellulose, a natural polysaccharide fiber. Synthetic fibers which are not derived from natural fibers can be derived from other natural sources or from mineral sources. Example synthetic fibers derived from natural sources include but are not limited to polysaccharides such as starch. Example fibers from mineral sources include but are not limited to polyolefin fibers such as polypropylene and polyethylene fibers, which are derived from petroleum, and silicate fibers such as glass and asbestos. Synthetic fibers are commonly formed, when possible, by fluid handling processes (e.g., extruding, drawing, or spinning a fluid such as a resin or a solution). Synthetic fibers are also formed by solid handling size reduction processes (e.g., mechanical chopping or cutting of a larger object such as a monolith, a film, or a fabric). Common synthetic fiber include but are not limited to nylon (polyamide), acrylic (polyacrylonitrile), aramid (aromatic polyamide), polyolefin (polyethylene and polypropylene), polyester, butadiene-stryene block copolymers, natural rubber, latex, and spandex (polyurethane).

Nonwoven materials are a type of fabric typically made from fibers in a web format. Nonwoven webs are described by Butler I, Batra SK, et al, Nonwovens Fabrics Handbook, Association of the Nonwoven Fabrics Industry, 1999, and by Vaughn EA, Nonwoven Fabric Sampler and Technology Reference, Association of the Nonwoven Fabrics Industry.

Nonwoven webs can be formed by direct extrusion processes during which the fibers and webs are formed at about the same point in time, or by preformed fibers which can be laid into webs at a distinctly subsequent point in time. Example direct extrusion processes include but are not limited to: spunbonding, meltblowing, solvent spinning, electrospinning, and combinations thereof typically forming layers. Example "laying" processes include wetlaying and drylaying. Example drylaying processes include but are not limited to airlaying, carding, and combinations thereof typically forming layers. Combinations of the above processes yield nonwovens commonly called hybrids or composites. Example combinations include but are not limited to spunbond-meltblown-spunbond (SMS), spunbond-carded (SC), spunbond-airlaid (SA), meltblown-airlaid (MA), and combinations thereof, typically in layers. Combinations which include direct extrusion can be combined at the about the same point in time as the direct extrusion process (e.g., spinform and coform for SA and MA), or at a subsequent point in time. In the above examples, one or more individual layers can be created by each process. For instance, SMS can mean a three layer, 'sms' web, a five layer 'ssmms' web, or any reasonable variation thereof wherein the lower case letters designate individual layers and the upper case letters designate the compilation of similar, adjacent layers.

In one optional embodiment of the present invention, the substrate may also comprise a layer comprising a liquid-absorbent material, such as those commonly used in disposable pull-on garments and other absorbent articles to such as comminuted wood pulp which is generally referred to as airfelt. Examples of other suitable absorbent materials, which may be present in the substrate in addition to or in place of the airfelt, include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

One optional embodiment of the present invention is directed to a disposable adsorbent article comprising the substrate. Non-limiting examples of disposable adsorbent articles include diapers, tampons, feminine pads, adult incontinence article, training pants, pull-up diapers, and the like. The substrate may comprise any portion or region of the disposable adsorbent articles, for example, the substrate may be topsheet of a diaper or a pad, or a portion thereof. Preferably when the substrate is incorporated into a disposable adsorbent article it will be a portion of the article which will contact water containing bodily wastes. Non-limiting examples of components of disposable adsorbent article which may comprise the substrate, include, topsheet, backsheet, adsorbent core, acquisition layers, corewrap, distribution layers, and the like. Additional illustrative, but non-limiting, information on construction, assembly, treatment and the various components of disposable diapers which may be the substrate of the present invention may be found in U.S. Pat. No. 3,860,003 to Buell; U.S. Pat. No. 5,151,092 to Buell; U.S. Pat. No. 5,221,274 to Buell; U.S. Pat. No. 5,554,145 to Roe et al. on September 10, 1996; U.S. Pat. No. 5,569,234 to Buell et al.; U.S. Pat. No. 5,580,411 to Nease et al.; U.S. Patent No. 6,004,306 to Robles et al.; U.S. Patent No. 5,938,648 to LaVon et al.; U.S. Pat. No. 5,865,823 to Curro; U.S. Pat. No. 5,571,096 to Dobrin et al.; U.S. Patent No. 5,518,801 to Chappell, et al.; U.S. Patent 4,573,986 to Minetola et al.; U.S. Pat. No. 3,929,135, to Thompson; U.S. Pat. No. 4,463,045 to Ahr, et al.; U.S. Pat. No. 4,609,518 to Curro et al.; U.S. Pat. No. 4,629,643 to Curro et al.; U.S. Pat. No. 5,037,416 to Allen et al.; U.S. Pat. No. 5,269,775 to Freeland et al.; U.S. Patent 4,610,678 to Weisman et al.; U.S. Patent 4,673,402 to Weisman et al.; U.S. Patent 4,888,231 to Angstadt; U.S. Pat. No. 5,342,338 to Roe; U.S. Pat. No. 5,260,345 to DesMarais et al.; U.S. Pat. No. 5,026,364 to Robertson; U.S. Patent 3,848,594 to Buell; U.S. Patent 4,846,815 to Scripps; U.S. Patent 4,946,527 to Battrell; U.S. Pat. No. 4,963,140 to Robertson et al.; U.S. Pat. No. 4,699,622 to Toussant et al.; U.S. Pat. No. 5,591,152 to Buell et al.; U.S. Patent 4,938,753 to Van Gompel, et al.; U.S. Patent No. 5,669,897 to LaVon, et al.; U.S. Patent No. 4,808,178 to Aziz et al.; U.S. Patent No. 4,909,803 to Aziz et al.; U.S. Pat. No. 4,695,278 to Lawson; U.S. Patent No. 4,795,454 issued to Dragoo; U.S. Pat. Nos. 5,607,760 to Roe on; U.S. Pat. No. 5,609,587 to Roe; U.S. Pat. No. 5,635,191 to Roe et al.; U.S. Pat. No. 5,643,588 to Roe et al.; U.S. Pat. No. 5,968,025 to Roe et al; U.S. Pat. No. 4,515,595 issued to Kievit et al; U.S. Pat. No. 5,330,458 issued to Buell et al; U.S. Pat. No. 2,075,189 issued to Galligan; U.S. Pat. No. 3,025,199 issued to Harwood; U.S. Pat. Nos. 4,107,364 and 4,209,563 issued to Sisson; U.S. Pat. No. 4,834,741 issued to Sabee; U.S. Pat. No. 5,190,563, issued to Herron; U.S. Pat. No. 5,234,423, issued to Young, et al; U.S. Pat. No. 5,147,345, issued to Young, et al; U.S. Pat. No. 6,443,940, issued to Ashton et al; U.S. Pat. No. 4,834,735, issued to Alemany et al; U.S. Pat. No. 4,988,344 issued to Reising, et al; U.S. Pat. No. 4,988,345 issued to Reising; and EP 0797968A1 (Kurt et al.) published on Oct. 1, 1997.

### EXAMPLES

### Example 1.

A coating composition is prepared by mixing petrolatum with Heavenscent SEA available from Haarmann & Reimer, Teterboro, NJ USA, in the ratio of approximately 5:1.

## Claims

1. A process for the delivery of a coating composition on to a substrate **characterized in that** said process comprises:
a) providing said coating composition, said coating composition comprising:
i) a starch encapsulated accord and
ii) a carrier; selected from the group consisting of alkoxylated nonionic surfactants, mineral oil, paraffin waxes, and combinations thereof.
wherein the weight ratio of said carrier to said starch encapsulated accord is equal to or greater than 1:1; and
b) delivering and attaching an effective amount of said composition to said substrate.

2. The process according to Claim 1 wherein said process is performed at a temperature greater than the melting point of said carrier.

3. The process according to any one of the preceding claims wherein said starch encapsulated accord has a particle size of from 0.5 µm to 1000 µm and an average particle size of from 1 µm to 300 µm; more preferably a particle size of from 1 µm to 500 µm and an average particle size of from 1 µm to 100 µm.

4. The process according to any one of the preceding claims wherein said coating composition is delivered to said substrate at a rate of from 1 grams per minute to 500 grams per minute.

5. The process according to any one of the preceding claims wherein process has a frequency of from 0 Hz to 50 Hz.

6. The process according to any one of the preceding claims wherein said coating composition is delivered to said substrate by a delivery system selected from the group consisting of bead applicators preferably having a diameter of greater than 200 µm, slot die coaters preferably having a slot with a width and length each independently greater than 200 µm, spray nozzles and combinations thereof.

7. A substrate prepared according to the process of any one of the preceding claims.

8. A disposable adsorbent article comprising the substrate of Claim 9.

## Patentansprüche

1. Verfahren für die Abgabe einer Beschichtungszusammensetzung auf ein Trägermaterial, **dadurch gekennzeichnet, dass** das Verfahren Folgendes umfasst:
a) das Bereitstellen der Beschichtungszusammensetzung, wobei die Beschichtungszusammensetzung Folgendes umfasst:
i) eine stärkeverkapselte Duftnote und
ii) einen Träger, ausgewählt aus der Gruppe bestehend aus alkoxylierten nichtionischen Tensiden, Mineralöl, Paraffinwachsen und Kombinationen davon.
wobei das Gewichtsverhältnis von Träger zu stärkeverkapselter Duftnote gleich oder größer als 1:1 ist; und
b) das Abgeben und Anbringen einer wirksamen Menge der Zusammensetzung an das Substrat.

2. Verfahren nach Anspruch 1, wobei das Verfahren bei einer Temperatur ausgeführt wird, die höher ist als der Schmelzpunkt des Trägers.

3. Verfahren nach einem der vorstehenden Ansprüche, wobei die stärkeverkapselte Duftnote eine Teilchengröße von 0,5 µm bis 1000 µm aufweist und eine durchschnittliche Teilchengröße von 1 µm bis 300 µm; mehr bevorzugt eine Teilchengröße von 1 µm bis 500 µm und eine durchschnittliche Teilchengröße von 1 µm bis 100 µm.

4. Verfahren nach einem der vorstehenden Ansprüche, wobei die Beschichtungszusammensetzung an das Substrat in einer Rate von 1 Gramm pro Minute bis 500 Gramm pro Minute abgegeben wird.

5. Verfahren nach einem der vorstehenden Ansprüche, wobei das Verfahren eine Frequenz von 0 Hz bis 50 Hz aufweist.

6. Verfahren nach einem der vorstehenden Ansprüche, wobei die Beschichtungszusammensetzung an das Substrat durch ein Abgabesystem ausgewählt aus der Gruppe bestehend aus Raupenauftragsgeräten, die vorzugsweise einen Durchmesser von über 200 µm aufweisen, Schlitzdüsenbeschichter, die vorzugsweise einen Schlitz mit einer Breite und Länge aufweisen, die jeweils unabhängig voneinander mehr als 200 µm betragen, Sprühdüsen und Kombinationen davon abgegeben wird.

7. Substrat, das nach dem Verfahren einer der vorstehenden Ansprüche hergestellt wird.

8. Einweg-Adsorptionsartikel, der das Substrat aus Anspruch 9 umfasst.

## Revendications

1. Procédé pour la libération d'une composition de revêtement sur un substrat **caractérisé en ce que** ledit procédé comprend :
a) la fourniture de ladite composition de revêtement, ladite composition de revêtement comprenant :
i) un accord encapsulé dans de l'amidon et
ii) un véhicule ; choisi dans le groupe constitué d'agents tensioactifs non ioniques alcoxylés, une huile minérale, des cires de paraffine, et leurs combinaisons.
dans lequel le rapport pondéral dudit véhicule sur ledit accord encapsulé dans de l'amidon est égal ou supérieur à 1:1 ; et
b) la libération et la fixation d'une quantité efficace de ladite composition sur ledit substrat.

2. Procédé selon la revendication 1, où ledit procédé est effectué à une température supérieure au point de fusion dudit véhicule.

3. Procédé selon l'une quelconque des revendications précédentes, dans lequel ledit accord encapsulé dans de l'amidon a une taille des particules allant de 0,5 µm à 1000 µm et une taille moyenne des particules allant de 1 µm à 300 µm ; plus préférablement une taille des particules allant de 1 µm à 500 µm et une taille moyenne des particules allant de 1 µm à 100 µm.

4. Procédé selon l'une quelconque des revendications précédentes, dans lequel ladite composition de revêtement est libérée audit substrat à une vitesse allant de 1 gramme par minute à 500 grammes par minute.

5. Procédé selon l'une quelconque des revendications précédentes, où le procédé a une fréquence allant de 0 Hz à 50 Hz.

6. Procédé selon l'une quelconque des revendications précédentes, dans lequel ladite composition de revêtement est libérée audit substrat par un système de libération choisi dans le groupe constitué d'applicateurs à bille ayant de préférence un diamètre supérieur à 200 µm, enrobeuses à filière plate ayant de préférence une fente avec une largeur et une longueur chacune indépendamment supérieure à 200 µm, buses de pulvérisation et leurs combinaisons.

7. Substrat préparé selon le procédé selon l'une quelconque des revendications précédentes.

8. Article adsorbant jetable comprenant le substrat selon la revendication 9.
